# EUROPÄISCHE PATENTSCHRIFT

(11) **EP 0 825 509 B1**
(45) Veröffentlichungstag und Bekanntmachung des Hinweises auf die Patenterteilung: **26.06.2002**
(21) Anmeldenummer: 97113600.7
(22) Anmeldetag: 06.08.1997
(51) Int. Cl.: G05D 3/20, G05B 19/35, A61B 6/14

(54) **Einrichtung zur präzisen Verstellung eines beweglich angeordneten Gerätes mittels eines elektromotorischen Antriebs**
Precise positioning device for a manoeuvrable apparatus using electric motor drive
Positionnement précis d'un appareil manoeuvrable à l'aide d'un entraînement à moteur électrique

(30) Priorität: 19.08.1996 DE 19633356
(43) Veröffentlichungstag der Anmeldung: 25.02.1998
(73) Patentinhaber: Sirona Dental Systems GmbH, 64625 Bensheim (DE)
(72) Erfinder: Pruss, Malte, Dipl.-Ing., 64665 Alsbach-Hähnlein (DE)
(74) Vertreter: Sommer, Peter

(56) Entgegenhaltungen:
- EP-A- 0 499 595
- US-A- 4 052 676
- US-A- 4 112 291
- US-A- 4 315 199
- US-A- 4 368 412
- US-A- 4 503 374
- US-A- 4 733 144
- US-A- 5 233 279

## Beschreibung

Die Erfindung bezieht sich auf eine Einrichtung zur präzisen Verstellung eines beweglich angeordneten Gerätes mittels eines elektromotorischen Antriebs mit Mitteln zur Erfassung zeit- und wegabhängiger Größen, die in einer Auswerteelektronik mit vorgegebenem Sollwertgrößen verglichen werden und deren Ausgangsgröße als Stellgröße für den Antriebsmotor dient.

Die Dokumente US 4 733 144 und US 4 112 291 zeigen den einschlägigen Stand der Technik.

Bei einer Vielzahl von Anwendungen ist es erwünscht, Geräte sehr präzise entlang eines bestimmten Verstellweges bewegen bzw. positionieren zu können. Der Begriff "Geräte" soll hier im weitesten Sinne verstanden werden und sowohl komplette Geräte (Maschinen) als auch Teile davon umfassen.

Beispiele für in Frage kommende Anwendungen sind spanabhebende Werkzeugmaschinen, bei denen beispielsweise Bohr-, Fräs- oder Sägeschlitten mit einem präzisen Vorschub in bezug auf ein zu bearbeitendes Werkstück zu verstellen sind. Ähnliche Anwendungsfälle liegen auch in der Medizintechnik, insbesondere Zahnmedizintechnik vor. Bei der computergesteuerten Rekonstruktion von Zahnpaßkörpern wird beispielsweise der Paßkörper mit Hilfe von zunächst genau positionierten und dann nach einem erstellten Arbeitsplan mit elektromotorischen Antrieben exakt verstellbaren Werkzeugen aus einem Werkstückblock herausgearbeitet.

Ein anderes Anwendungsbeispiel, auf das in der Beschreibung des nachfolgend erläuterten Ausführungsbeispiels Bezug genommen wird, betrifft die zahnärztliche Röntgentechnik, wo man zur Erstellung von CEPH-Aufnahmen vom Kopf eines Patienten die Höhenverstelleinrichtung der CEPH-Anlage und die Kopfhalterung für den Patienten exakt zueinander positionieren bzw. einstellen muß.

Bei den vorgenannten Anwendungsbeispielen ist stets die Position (Weg), die Geschwindigkeit und Beschleunigung der zu verstellenden Geräte zu erfassen.

Zur genauen Verstellung solcher Geräte setzte man bisher entweder Schrittmotore ein oder führt die Verstellung mit Hilfe eines Wegaufnehmers und eines Tachogenerators mit entsprechender Auswerteelektronik durch.

Schrittmotore, die pro Puls einen bestimmten, fest eingestellten Winkelschritt ausführen, typischerweise 0,9 oder 1,8 Winkelgrade, sind zwar vergleichsweise preiswert zu haben, aber teurer als DC-Motoren. Sie sind jedoch mit dem Nachteil behaftet, daß sie nur bei konstanter Frequenz exakt arbeiten. Bei Frequenzänderungen, z. B. bei Beschleunigung oder Verzögerung unter Last, ändert sich die Stellkraft und damit der Verstellweg, so daß keine ausreichend genaue Positionierung des Gerätes möglich ist.

Die heute standardmäßig eingesetzten Lageregelungen über einen mit der Motorwelle gekuppelten Wegaufnehmer und einem Tachogenerator sind, da sie mehrere Teile erfordern, vergleichsweise aufwendig. Dies ist insbesondere dann der Fall, wenn man zur exakten Positionierung bzw. Verstellung die Geschwindigkeit (v), die Beschleunigung (a) und die Position (s) des Gerätes bestimmen will.

Für die Positionierung der Strahlenquelle und des korrespondierend dazu angeordneten Bildaufnehmers würde dies bedeuten, daß sowohl Wegaufnehmer für die Sollwertvorgaben als auch Wegaufnehmer für die Stellmotoren vorzusehen wären. Dabei würde ein Sensor für die Erfassung der Position (Weg) und ein weiterer Sensor für die Erfassung der Geschwindigkeit bzw. Drehzahl des Motors benötigt werden, aus denen die Beschleunigungswerte zusätzlich zu ermitteln wären. Die Signale müßten in mehrkreisigen Regelschleifen verarbeitet werden, was einen erhöhten Mehraufwand an Elektronik und Software bedeuten würde.

Der Erfindung liegt die Aufgabe zugrunde, eine Einrichtung der eingangs genannten Gattung anzugeben, mit der sich eine einfachere und preiswertere, aber sehr exakte Verstellung bzw. Positionierung eines Gerätes erzielen läßt. Diese Aufgabe wird erfindungsgemäß durch die merkmale des Anspruchs 1 gelöst.

Bei der erfindungsgemäß vorgeschlagenen Lösung wird lediglich ein an sich beliebiger, vorzugsweise jedoch ein besonders preisgünstiger DC-Motor und ein Encoder (Pulsgeber), der auf der Motorachse aufgesetzt ist, benötigt, um alle drei Bewegungsgrößen (v, a, s) abzuleiten. Die im Inkrementalgeber des Encoders in digitaler Form vorliegenden Größen brauchen nicht in analoge Signale umgewandelt zu werden. Somit ist eine direkte Implementierung unter anderem auch in Software möglich. Die im Encoder erfaßten Signale werden direkt in eine Auswerteelektronik gegeben und mit Sollwerten für die drei Bewegungsgrößen v, a, s verglichen. Die Auswerteelektronik, die die Funktion eines Summierers erfüllt, errechnet hieraus direkt eine Stellgröße für den Motor, die über einen Verstärker mit Integrationsverhalten dem Motor zugeführt wird.

Anhand der Zeichnung werden zwei Varianten der Erfindung näher erläutert. Es zeigen:
- Fig. 1: ein zahnärztliches Röntgendiagnostikgerät zur Erstellung von CEPH-Aufnahmen vom Kopf eines Patienten,
- Fig. 2: die für CEPH-Aufnahmen vorgesehene Vorrichtung in einer schaubildlichen Darstellung,
- Fig. 3: die Vorrichtung nach Fig. 2 in einer Frontansicht, teilweise im Schnitt,
- Fig. 4: ein erstes Ausführungsbeispiel der Erfindung,
- Fig. 5: ein zweites Ausführungsbeispiel der Erfindung und
- Fig. 6: ein Impulsfolgediagramm

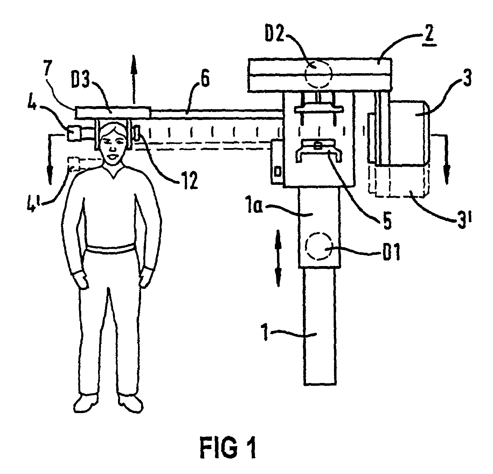

Die Fig. 1 zeigt in einer Prinzipdarstellung ein zahnärztliches Röntgendiagnostikgerät zur Erstellung von Panorama-Schichtaufnahmen und sogenannten CEPH-Aufnahmen vom Kopf eines Patienten. Das Gerät enthält eine Tragsäule 1, an der eine Dreheinheit 2 gehaltert ist, die Träger einerseits einer Röntgenstrahlenquelle 3 und andererseits in einem definierten Abstand dazu angeordneten Röntgenzeilenkamera 4. Mit 5 ist eine (erste) Kopfhalte- und Positioniereinrichtung bezeichnet, mit der in bekannter Weise der Patientenkopf zur Erstellung von Panorama-Schichtaufnahmen in einer definierten Position fixiert werden kann.

Die Tragsäule 1 enthält ein höhenverstellbares Teil 1a, das in der angegebenen Pfeilrichtung mittels eines symbolisch angedeuteten Antriebsmotors D1 höhenverstellbar ausgebildet ist. Die Dreheinheit 2 weist mindestens einen weiteren Antrieb D2 auf, mit dem sie in bekannter Weise gedreht und geschwenkt werden kann, um die zuvor erwähnte Panorama-Schichtaufnahme erstellen zu können.

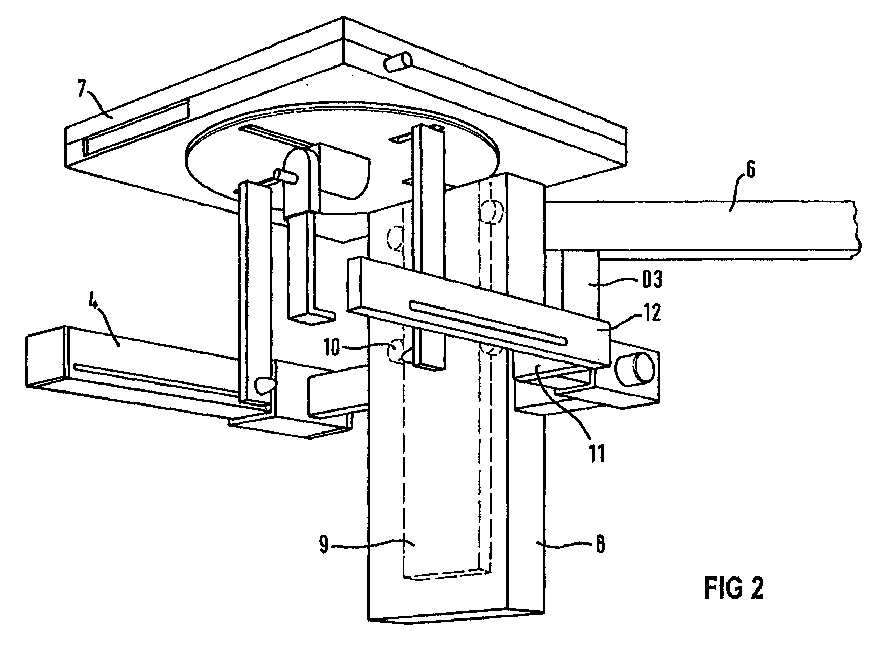

Am höhenverstellbaren Teil 1a der Tragsäule 1 ist ein Auslegerarm 6 befestigt, der eine (zweite) Kopfhalte- und Positioniereinrichtung 7 trägt. Diese ist in Fig. 2 in schaubildlicher Darstellung dargestellt. Der Auslegerarm 6 umfaßt ein Gehäuse 8, an dem ein Schwert 9, welches die Kopfhalte- und Positioniereinrichtung trägt, mit Hilfe von im Gehäuse angeordneten Führungsrollen 10 verstellbar gelagert ist. Die Zeilenkamera 4, die einen röntgenstrahlenempfindlichen Aufnahmesensor beinhaltet, ist mittels eines Querträgers 11 mit einer Vorblende 12 verbunden, die dazu dient, den an sich schon in bekannter Weise durch eine im Bereich der Röntgenstrahlenquelle 3 benachbart angeordnete Sekundärblende begrenzten Fächerstrahl nochmals exakt auf die Schlitzbreite und Schlitzlänge der Zeilenkamera 4 zu justieren.

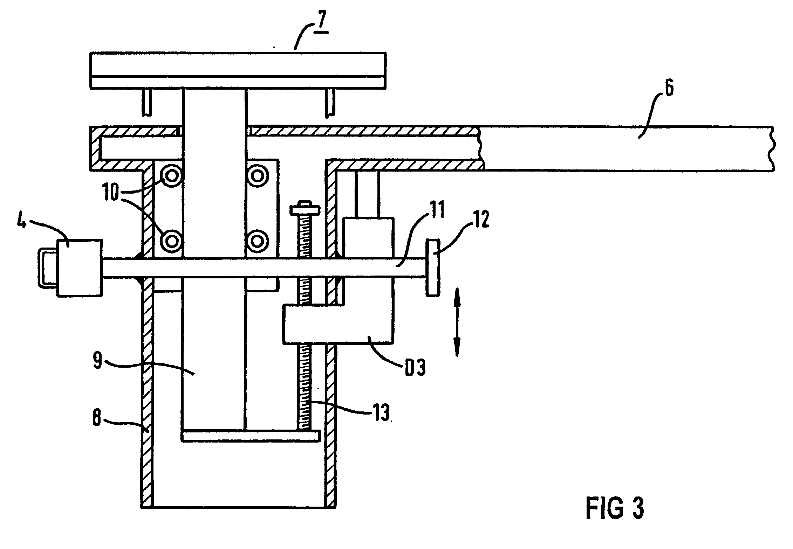

Anhand der Fig. 3, die die Vorrichtung in Frontansicht und teilweise im Schnitt zeigt, wird der Bewegungsablauf erläutert, der mittels der erfindungsgemäßen Einrichtung exakt vollzogen werden soll.

Am Schwert 9, welches die gesamte Kopfhalte- und Positioniereinrichtung trägt, befindet sich eine Gewindespindel 13, die zusammen mit einem allgemeinen mit D3 bezeichneten Getriebemotor zusammenwirkt. Der Getriebemotor D3 ist entweder am Gehäuse 8 oder am Tragarm 6 befestigt. Mit Hilfe der aufgezeigten Verstellanordnung soll erreicht werden, daß, wenn die Röntgenstrahlenquelle 3 zusammen mit der Zeilenkamera 4 in der Vertikalen bewegt wird, dann die Kopfhalte- und Positioniereinrichtung 7 ortsfest im Raum gehalten wird, so daß diese während der CEPH-Aufnahme effektiv keine Bewegung ausführt.

Eine CEPH-Aufnahme läuft in Slot-Technik ab. Der Kopf eines stehenden (oder sitzenden) Patienten wird je nach Anordnung der Zeilenkamera 4 von oben nach unten (bei waagerechter Anordnung) oder von links nach rechts (bei senkrechter Anordnung) mit fächerartig gebündelten Röntgenstrahlen beaufschlagt. Die Strahlen treffen, justiert durch die bereits vorgenannte Vorblende 11, genau auf den Schlitz in der Zeilenkamera, hinter dem der eigentliche CCD-Sensor angeordnet ist. Mit Hilfe des Antriebs D1 verfährt das gesamte Gerät, also Strahlenquelle 3 und Zeilenkamera 4 gemeinsam von einer Ausgangsposition aus in der Vertikalen. Gleichzeitig wird die Kopfhalte- und Positioniereinrichtung 7 mit Hilfe des Antriebs D3 in Gegenrichtung gefahren, wobei die beiden gegenläufigen Bewegungen so aufeinander abgestimmt sein müssen, daß der Patientenkopf räumlich fixiert, d. h. ortsfest, verbleibt. Selbst geringe Abweichungen einer nicht antagonistischen Bewegung würde der Patient sofort als unangenehm empfinden. Daraus folgt, daß die Bewegungsgrößen (Weg, Geschwindigkeit und Beschleunigung) absolut synchron erfolgen müssen.

Anhand der nachfolgenden Figuren wird die erfindungsgemäße impulsäquivalente Regelung am vorerläuterten Beispiel mit den Antriebsmotoren D1 und D3 zur exakten Verstellung des Tragteils 1a und der Kopfhalte- und Positioniervorrichtung 7 näher erläutert, wobei angenommen wird, daß das höhenverstellbare Tragteil 1a mit dem Verstellmotor D1 den Sollwert für die Position (Weg s), die Geschwindigkeit (v) und die Beschleunigung (a) vorgibt.

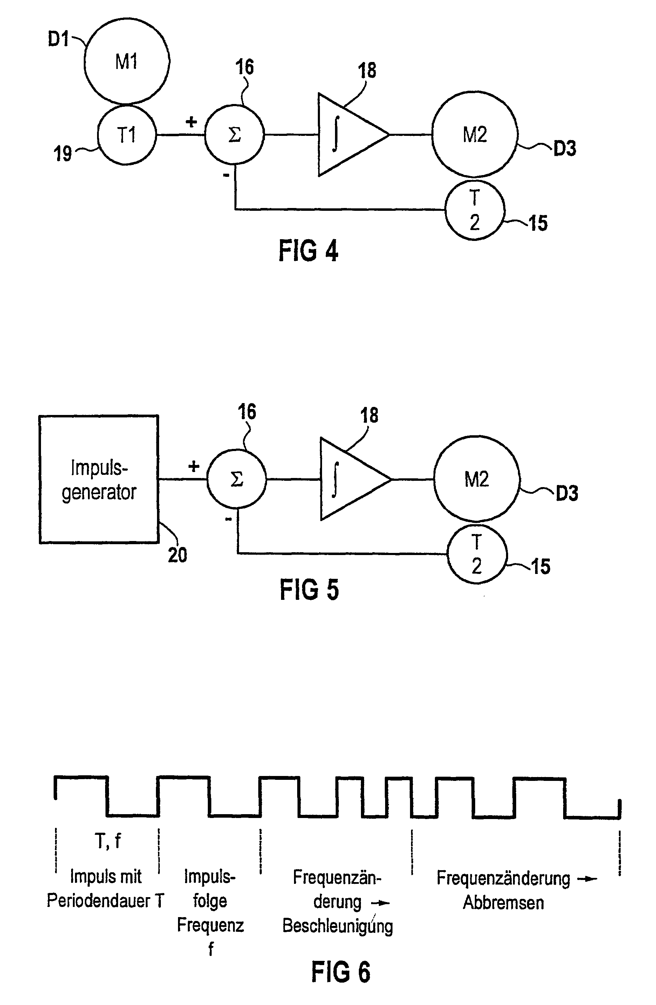

Der Antriebsmotor D3 ist mit einem Encoder 15 (Pulsgeber) gekoppelt, der pro Umdrehung eine bestimmte Anzahl von Impulsen liefert. Der Encoder 15 enthält standardmäßig einen geeigneten Inkrementalgeber, z.B. eine Lichtschranke, die mit auf der Motorwelle aufgesetzter Schlitzscheibe zusammenwirkt, der pro Umdrehung eine Anzahl von Impulsen erzeugt. Die generierten Impulse werden als Istwerte einer Auswerteelektronik 16 zugeführt. Die Sollwerte können auf verschiedene Weise generiert werden. Im dargestellten Anwendungsfall nach Figur 4 werden sie vom Antriebsmotor D1 (dem Antriebsmotor für das höhenverstellbare Tragteil 1a) und einem mit diesem gekuppelten Encoder 19 generiert. Der Encoder 15 des Antriebsmotors D3 liefert, wie erläutert, eine den Istwerten entsprechende Folge von Impulsen. Die Auswerteelektronik 16 hat die Funktion eines Summierers, der die Sollwertimpulse aus dem Encoder 19 und die Istwertimpulse aus dem Encoder 15 addiert oder subtrahiert. Ein Richtungsseparator bestimmt, ob die Sollwertimpulse aus dem Encoder 19 oder Istwertimpulse aus dem Encoder 15 addiert bzw. subtrahiert werden. Der Summierer wird dabei von den Sollwertimpulsen bzw. den Impulsen aus dem Encoder 15 getaktet. Eine besonders einfache Lösung besteht aus einem UP-Down-Counter, von dem das oberste Bit als Ausgang benutzt wird. Die Ausgangsimpulse werden auf einen Verstärker 18 mit Tiefpaß- bzw. Integrationsverhalten gegeben, der den Antriebsmotor D3 ansteuert. Ein Puls als vorgegebener Sollwert dreht den Motor so weit, bis von dem mit dem Motor gekuppelten Encoder 15 ein Puls zurückgeliefert wird. Ein Puls entspricht somit exakt einer Weglänge s. Die Pulsfolge beschleunigt entsprechend dem Motor. Ist z. B. die Impulsfolge, die vom Encoder 19 für den Sollwert generiert wird, größer, wird der Motor D3 so lange beschleunigt, bis die Impulsfolgen wieder übereinstimmen. Die Genauigkeit der Positionierung hängt von dem Teilungsverhältnis von Sollwert-Pulsfolge zu Istwert-Pulsfolge ab. Dieses Teilungsverhältnis kann im Summierer festgelegt sein. Der Summierer kann sowohl in Software als auch in Hardware realisiert sein.

Im zweiten Ausführungsbeispiel gemäß Figur 5 werden die Sollwerte von einem Impulsgenerator 20 erzeugt. Die Ausgangswerte des Summierers 16 werden auch hier wieder über den Verstärker 18 mit Integratorverhalten direkt dem Antriebsmotor D3 zugeführt.

Wichtig ist das Integrationsverhalten des Verstärkers 18. Am einfachsten läßt sich dies durch ein RC-Glied vor dem Eingang des Verstärkers realisieren. Dieser Integrationsverstärker stellt den eigentlichen Regler dar, welcher als Tiefpaß die digitalen Impulse für eine analoge Motorbrücke aufbereitet.

In Fig. 6 ist eine Impulsfolge dargestellt, anhand derer erklärt wird, wie die Größen für Weg (s), Geschwindigkeit (v) und Beschleunigung (a) generiert werden.

Die Flanke eines Impulses mit der Periodendauer (T) repräsentiert eine feste Weglänge, die der Motor bzw. das von ihm angetriebene Gerät, ähnlich einem Winkelschritt eines Schrittmotors, zurücklegen muß. Eine Pulsfolge mit einer Frequenz (f) repräsentiert die Drehzahl des Motors und legt damit die Geschwindigkeit (v) fest. Eine Änderung der Frequenz (Δf) generiert somit eine Stellgröße für die Beschleunigung (a). Die gesamte Weglänge (s), die der Antriebsmotor D3 und damit das mit ihm gekuppelte Gerät zurücklegen muß, ergibt sich im Ausführungsbeispiel aus den aufgezeigten sieben Impulsen. Aus der ersichtlichen Frequenzänderung ergibt sich, daß der Motor kurz beschleunigt und danach wieder abgebremst wird. Der gesamte zurückgelegte Weg ist somit allein abhängig von den Impulsen pro Umdrehungen, die der Encoder, der mit der Motorwelle des Antriebsmotors D3 fest gekoppelt ist, erzeugt. Je mehr Impulse pro Umdrehung geliefert werden, desto feiner wird die Auflösung für die Weglänge. Wenn der Sollwert durch einen separaten Impulsgenerator vorgegeben wird, kann die Vorgabe durch abgespeicherte Programme oder durch Software erfolgen.

Obgleich es an sich möglich ist, jeden geeigneten Verstellmotor einzusetzen, ist es vorteilhaft, einen DC-Motor mit integriertem Encoder einzusetzen.

## Patentansprüche

1. Einrichtung zur präzisen Verstellung eines beweglich angeordneten Gerätes mittels eines elektromotorischen Antriebes, wobei der Einrichtung vorgebbare Sollwertimpulse zugeführt werden, wobei die Einrichtung einen Antriebsmotor (D3), einen mit diesem gekoppelten, Istwertimpulse erzeugenden Encoder (15) und eine Auswerteelektronik (16) enthält, und wobei die Auswerteelektronik (16) die Funktion eines Summierers erfüllt, in welchem die Istwertimpulse addiert und die Sollwertimpulse subtrahiert werden oder die Sollwertimpulse addiert und die Istwertimpulse subtrahiert werden, **dadurch gekennzeichnet, daß** das Ausgangssignal der Auswerteelektronik (16) unmittelbar einem Verstärker (18) mit Integrationsverhalten zugeführt wird, der den Antriebsmotor (D3) direkt ansteuert.

2. Einrichtung nach Anspruch 1, **dadurch gekennzeichnet, daß** der Summierer als Up-Down-Counter ausgeführt ist, und daß das Ausgangssignal der Auswerteelektronik (16) das oberste Bit des Up-Down-Counters ist.

3. Einrichtung nach Anspruch 1 oder 2, **dadurch gekennzeichnet, daß** der Summierer in Software realisiert ist.

4. Einrichtung nach einem der Ansprüche 1 bis 3, **dadurch gekennzeichnet, daß** sie einen Richtungsseparator aufweist, der festlegt, ob die Istwertimpulse oder Sollwertimpulse addiert bzw. subtrahiert werden.

5. Einrichtung nach einem der Ansprüche 1 bis 4, **dadurch gekennzeichnet, daß** die Integration durch ein RC-Glied im Integrationsverstärker (18) erfolgt.

6. Einrichtung nach einem der Ansprüche 1 bis 5, **dadurch gekennzeichnet, daß** die Sollwertimpulse von einem Impulsgenerator (20) generiert werden.

7. Einrichtung nach einem der Ansprüche 1 bis 5, **dadurch gekennzeichnet, daß** die Sollwertimpulse von einem separaten Motor (D1) mit gekoppeltem Encoder (19) generiert werden.

8. Einrichtung nach einem der Ansprüche 1 bis 6, **dadurch gekennzeichnet, daß** die Sollwertimpulse im Datenspeicher eines Mikrokontrollers abgelegt sind.

## Claims

1. Mechanism for the precise adjustment of a movably arranged device by means of an electromotive drive, the mechanism being fed predeterminable set-value impulses, the mechanism comprising a drive motor (D3), an encoder (15), coupled to the latter and generating actual-value pulses, and an evaluation electronics unit (16), and the evaluation electronics unit (16) performing the function of an adder, in which the actual-value pulses are added and the set-value pulses are subtracted or the set-value pulses are added and the actual-value pulses are subtracted, **characterized in that** the output signal of the evaluation electronics unit (16) is fed directly to an amplifier (18) with integrating action, which directly activates the drive motor (D3).

2. Mechanism according to Claim 1, **characterized in that** the adder is configured as an up-down counter, and **in that** the output signal of the evaluation electronics unit (16) is the uppermost bit of the up-down counter.

3. Mechanism according to Claim 1 or 2, **characterized in that** the adder is implemented in software.

4. Mechanism according to one of Claims 1 to 3, **characterized in that** it has a direction separator, which fixes whether the actual-value pulses or set-value pulses are added or subtracted.

5. Mechanism according to one of Claims 1 to 4, **characterized in that** the integration is performed by an RC element in the integrating amplifier (18).

6. Mechanism according to one of Claims 1 to 5, **characterized in that** the set-value pulses are generated by a pulse generator (20).

7. Mechanism according to one of Claims 1 to 5, **characterized in that** the set-value pulses are generated by a separate motor (D1) with a coupled encoder (19).

8. Mechanism according to one of Claims 1 to 6, **characterized in that** the set-value pulses are stored in the data memory of a microcontroller.

## Revendications

1. Dispositif destiné au réglage précis d'un appareil au moyen d'un entraînement électromotrice, lequel dispositif est disposé de façon mobile et équipé de moyens pour enregistrer des grandeurs dépendant du temps et du parcours, auquel dispositif sont amenés des impulsions de valeurs de consigne susceptibles d'être prédéterminées ; lequel dispositif comporte un moteur d'entraînement (D3), un encodeur (15) couplé à ce moteur et générant des impulsions de valeurs réelles et une électronique d'évaluation (16); l'électronique d'évaluation (16) remplit la fonction d'un additionneur dans lequel les impulsions de valeurs réelles sont additionnées et les impulsions de valeurs de consigne sont soustraites ou les impulsions de valeurs de consigne sont additionnées et les impulsions de valeurs réelles sont soustraites, **caractérisé en ce que** le signal de sortie de l'électronique d'évaluation (16) est amené directement vers un amplificateur (18) avec à comportement d'intégration qui commande directement le moteur d'entraînement (D3).

2. Dispositif selon la revendication 1, **caractérisé en ce que** l'additionneur est réalisé comme compteur up-down et **en ce que** le signal de sortie de l'électronique d'évaluation (16) est le bit le plus haut du compteur up-down.

3. Dispositif selon la revendication 1 ou 2, **caractérisé en ce que** l'additionneur est réalisé sous forme de logiciel informatique.

4. Dispositif selon une des revendications 1 à 3, **caractérisé en ce qu'**il comporte un séparateur de direction qui détermine si les impulsions de valeurs réelles ou les impulsions de valeur de consigne sont additionnées ou soustraites.

5. Dispositif selon une des revendications 1 à 4, **caractérisé en ce que** l'intégration est effectuée par un élément RC dans l'amplificateur d'intégration (18).

6. Dispositif selon une des revendications 1 à 5, **caractérisé en ce que** les impulsions de valeur de consigne sont générées par un générateur d'impulsions (20).

7. Dispositif selon une des revendications 1 à 5, **caractérisé en ce que** les impulsions de valeur de consigne sont générées par un moteur séparé (D1) avec un encodeur (19) couplé.

8. Dispositif selon une des revendications 1 à 6, **caractérisé en ce que** les impulsions de valeurs de consigne sont mémorisées dans la mémoire de données d'un microcontrolleur.
